(19) Europäisches Patentamt
European Patent Office
Office européen des brevets

(11) Publication number: 0 111 841
B1

(12) EUROPEAN PATENT SPECIFICATION

(45) Date of publication of patent specification: 06.05.87

(51) Int. Cl.[4]: A 61 K 9/06, A 61 K 37/02

(21) Application number: 83112369.0

(22) Date of filing: 08.12.83

# (54) LHRH preparations for intranasal administration.

(30) Priority: 10.12.82 US 448548

(43) Date of publication of application:
27.06.84 Bulletin 84/26

(45) Publication of the grant of the patent:
06.05.87 Bulletin 87/19

(84) Designated Contracting States:
BE CH DE FR GB IT LI LU NL SE

(56) References cited:
FR-A-2 321 275
GB-A-1 454 105

(73) Proprietor: SYNTEX (U.S.A.) INC.
3401 Hillview Avenue P.O. Box 10850
Palo Alto California 94303 (US)

(72) Inventor: Anik, Shabbir Tyabji
724 Arastradero Road 313
Palo Alto California 94306 (US)

(74) Representative: Barz, Peter, Dr. et al
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

EP 0 111 841 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel LHRH formulation. More particularly, the present invention relates to an aqueous LHRH active nona- or decapeptide preparation containing a surfactant derived from a bile acid or a salt thereof which is suitable for intranasal administration.

Luteinizing hormone (LH) and follicular stimulating hormone (FSH) are released from the anterior pituitary gland under the control of the releasing hormone LHRH produced in the hypothalmic reion. LH and FSH act on the gonads to stimulate the synthesis of steroids hormones and to stimulate gamete maturation. The pulsatile release of LHRH, and thereby the release of LH and FSH, control the reproductive cycle in domestic animals and humans. LHRH also effects the placenta, and the gonads indirectly in causing the release of chorionic gonadotropin (hCG).

LHRH peptides have heretofore been administered almost exclusively by injection. Other methods of administration, e.g., oral, nasal, intratracheal and rectal administration, have been investigated but because these compounds are polypeptides, these routes of administration have had little or no pharmacological effect because of poor absorption or the effect has been somewhat uncontrollable at best because of irregular absorption. When non-injeciton routes are used, the drug dose must be substantially increased because the amount of drug reaching the intended site of activity is greatly reduced by mechanisms ongoing in the absorption process which prevent absorption or degrade the peptides during absorption.

GB—A—1 454 105 discloses the use of LHRH analogs in nasal preparations which may be in the form of oily suspensions, fat-containing ointments or isotonic aqueous solutions. The oily suspensions and fat-containing ointments, optionally, contain a surface-active agent in order to reduce the surface tension thereof.

The nasal administration of insulin to rats is described by S. Hirai, et all, in the *International Journal of Pharmaceutics,*, 9, 165—172 (1981). In this reference, aqueous insulin solutions containing a surfactant have indicated that, in certain instances, the presence of certain surfactants result in decreased serum glucose levels over control solutions comprising water and insulin. The Hirai reference discloses surfactants from a broad range of types including ethers, esters, anionic surfactants, amphoterics, bile acid salts, a glycoside and a peptidelipid. Except for a few isolated cases, the polyoxethylene fatty acid ethers, anionic, amphoteric, bile acid salt, gyloside, and peptidelipid surfactants all showed an approximately equivalent decrement in plasma glucose levels. While the decrement in plasma glucose levels is an indirect measure of insulin absorption, it is clear that most of the surfactants tested had an equal effect on the uptake of insulin administered by the nasal route in rats.

GB—A—1 527 605 (corresponding to FR—A—2 321 275) describes the use of surfactants for enhancing insulin uptake across the nasal membrane. Hirai is one of the inventors on this patent. The effect of sodium glycocholate onthe uptake of insulin administered by nasal spray to humans is reported by A.E. Poteroli, et al, *British Medical Journal,* Vol 284, pp 303—306, (1982). Since a single surfactant was used there the relative effect of sodium glycocholate versus other surfactant on insulin uptake cannot be apprised.

It has now been found that the combination of bile acids and their pharmaceutically acceptable salts with LHRH analogs greatly enhance LHRH absorption across the nasal membranes relative to other surfactants.

The present invention provides a nasal composition comprising a nona- or decapeptide or its pharmaceutically acceptable salt having LHRH agonist or antagonist activity, and a surfactant, which composition is characterized in that it is an aqueous formulation containing, as surfactant, bile acid or a pharmaceutically acceptable salt thereof.

In one embodiment the present invention relates to a novel nasal spray composition comprising an LHRH agonist or antagonist compound plus a surfactant which is a bile acid or a pharmaceutically acceptable salt thereof formulated in an aqueous solution which may be buffered and may contain other appropriate pharmaceutical excipients, for example, co-solvents, chelating agents and preservatives.

The invention also relates to a method of preparing a medicament for enhancing the nasal uptake of LHRH analogs, which method comprises adding a surfactant which is a bile acid or a pharmaceutically acceptable salt thereof to a nasal composition comprising a nona- or decapeptide or a pharmaceutically acceptable salt thereof having LHRH agonist or antagonist activity formulated in an aqueous solution.

Agonist and antagonist analogs of LHRH have been prepared and found to be useful in the control of fertility in both male and female; are useful in the reduction in accessory organ weight in male and female; will promote weight gain in domesitic animals in feedlot situations; will stimulate abortion in pregnant animals; and in general act as chemical sterilants.

The natural hormone releasing hormone, LHRH, is a decapeptide comprised of naturally occurring amino acids (which have the L-configuration except for the achiral amino acid glycine). Its sequence is as follows:

$$\underset{1}{(pyro)Glu}\text{-}\underset{2}{His}\text{-}\underset{3}{Trp}\text{-}\underset{4}{Ser}\text{-}\underset{5}{Tyr}\text{-}\underset{6}{Gly}\text{-}\underset{7}{Leu}\text{-}\underset{8}{Arg}\text{-}\underset{9}{Pro}\text{-}\underset{10}{GlyNH_2}$$

A large number of analogs of this natural material have been prepared and studied in attempts to find compounds which have greater agonist or antagonist activity.

By far the most significant modification for the enhancement of agonist activity is obtained by changing the 6-position residue from Gly to a D-amino acid. In addition, substantial increased agonist activity is obtained by elminating the Gly-$NH_2$ in position 10 to afford a nonapeptide as an alkyl, cycloalkyl or fluoroalkylamide or by replacing the Gly-$NH_2$ by an α-azaglycine amide. In yet other instances modifications have been made at positions 1 and 2 in attempts to enhance agonist activity.

In addition to the preparation of agonist analogs, a number of nona- and decapeptides have been prepared which are competitive antagonists to LHRH, all of which require deletion or replacement of the histidine residue at position 2. In general, it appears that a D-amino acid placed in the sequence at that position gives the best antagonist activity. It has also been shown that adding a modification at the 6 position, which, without the modification at position 2 results in the agonist activity noted above, enhances the antagonist activity of the 2-modifed analogs. Additional increments in antagonist activity may be had by modifying positions 1, 3 and/or 10 in the already 2, 6 modified peptide. It has also been shown that N-acylation of the amino acid at position 1 is helpful.

The invention has application to LHRH and all synthetic agonist and antagonist analogs thereof. The patent and periodical literature is replete with nona- and decapeptides of this type. It is intended that all such compounds are embraced by this invention.

Nona- or decapeptides having LHRH agonist or antagonist activity are disclosed, along with processes for preparation thereof, in the following U.S. Patents. 3,813,382; 3,842,065; 3,849,389; 3,855,199; 3,886,135; 3,890,437; 3,892,723; 3,896,104; 3,901,872; 3,914,412; 3,915,947; 3,929,759; 3,937,695; 3,953,416; 3,974,135; 4,010,125; 4,018,914; 4,022,759; 4,022,760; 4,022,761; 4.024,248; 4,034,082; 4,072,668; 4,075,189; 4,075,192; 4,086,219; 4,101,538; 4,124,577; 4,124,578; 4,143,133; 4,234,571; 4,253,997; 4,292,313; 4,341,767.

This list is not intended to be exhaustive of all U.S. Patents covering LHRH analogs but does represent the majority; nor is this invention limited exclusively to the compounds disclosed in the recited patents.

Of the numerous LHRH analogs disclosed by the foregoing patents and in the literature in general, there are certain compounds which have been shown to be preferred for the control of fertility, enhancement or growth, treatment of prostatic cancer, for inducing abortion and other situations where LHRH agonists or antagonists have utility.

One such group of agonist compound is the group of LHRH analogs disclosed in U.S. Patent No. 4,234,571 and represented by the following formula:

$$\text{(pyro)Glu-His-V-Ser-W-X-Y-Arg-Pro-Z} \qquad \text{(I)}$$

and the pharmaceutically acceptable salts thereof wherein:

V is tryptophyl, phenylalanyl or 3-(1-naphthyl)-L-alanyl;

W is tyrosyl, phenylalanyl or 3-(1-pentafluorophenyl)-L-alanyl;

X is a D-amino acid residue

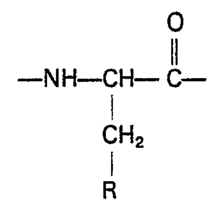

wherein R is

(a) a carbocyclic aryl-containing radical selected from naphthyl, anthryl, fluorenyl, phenanthryl, biphenylyl, benzhydryl and phenyl substituted with three or more straight chain lower alkyl groups; or

(b) a saturated carbocyclic radical selected from cyclohexyl substituted with three or more straight chain lower alkyl gorups, perhydronaphthyl, perhydrobiphenylyl, perhydro-2,2-diphenylmethyl and adamantyl;

Y is leucyl, isoleucyl, nor-leucyl or N-methyl-leucyl; Z is glycinamide or —NR—$R^1$, wherein $R^1$ is lower alkyl, cycloalkyl, fluoro lower alkyl or

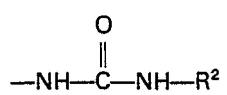

wherein $R^2$ is hydrogen or lower alkyl.

More preferred are those compounds of Formula I wherein V is tryptophyl or phenylalanyl; W is tyrosyl; X is 3-(2-naphthyl)-D-alanyl or 3-(2,4,6-trimethylhenyl)-D-alanyl; Y is leucyl or N-methyl-leucyl; and Z is glycinamide or -NHEt.

Particularly preferred compounds of Formula I are:

(pyro) Glu-His-Trp-Ser-Tyr-3-(2-naphthyl)-D-alanyl-Leu-Arg-Pro-Gly-$NH_2$;

(pyro) Glu-His-Trp-Ser-Tyr-3-(2-naphthyl)-D-alanyl-N-methyl-Leu-Arg-Pro-Gly-$NH_2$;

(pyro) Glu-His-Phe-Ser-Tyr-3-(2-naphthyl)-D-alanyl-Leu-Arg-Pro-Gly-$NH_2$

(pyro) Glu-His-Trp-Ser-Tyr-3-(2,4,6-trimethylphenyl)-D-alanyl-Leu-Arg-Pro-Gly-$NH_2$;

(pyro) Glu-His-Trp-Ser-Tyr-3-(2-(naphthyl)-D-alanyl-Leu-Arg-Pro-NHEt;

(pyro) Glu-His-Trp-Ser-Tyr-3-(2-naphthyl)-D-alanyl-N-methyl-Leu-Arg-Pro-NHEt; and

(pyro) Glu-His-Trp-Ser-Tyr-3-(2-naphthyl)-D-alanyl-Leu-Arg-Pro-Aza-Gly-$NH_2$; and their pharmaceutically acceptable salts.

Further particularly preferred agonist compounds from other noted U.S. Patents and reported in the periodical literature are:

(pyro) Glu-His-Trp-Ser-Tyr-D-Trp-Leu-Arg-Pro-Gly$NH_2$, Coy, C. D., *J. Med. Chem., 19,* 423 (1976),

(pyro) Glu-His-Trp-Ser-Tyr-D-Trp-N-Me-Leu-Arg-Pro-NHEt, Corbin, A. & Bex, F. J., "LHRH Peptides as Female and Male Contraceptives," Shelton, J. D. & Sciarra, J. J., Eds., Harper & Row, Philadelphia (1981), pp 68—84;

(pyro) Glu-His-Trp-Ser-Tyr-D-Trp-Leu-Arg-Pro-NHEt; Rivier, J. et al, "Peptides: Chemistry, Structure and Biology — Proceedings of the Fourth American Peptide Symposium," R. Walter & J. Meienhofer, Eds, (1975) Ann Arbor Science Publications, p. 863—870;

(pyro) Glu-His-Trp-Ser-Tyr-D-Ala-Leu-Arg-Pro-Gly$NH_2$, U.S. Patent 3,914,412;

(pyro) Glu-His-Trp-Ser-Tyr-D-Leu-Leu-Arg-Pro-NHEt; Fujino, M. et al, *Biochem. Biophys. Res. Commun.,* 60, 406 (1974);

(pyro) Glu-His-Trp-Ser-Tyr-D-Leu-Leu-Arg-Pro-Gly$NH_2$, U.S. Patent 3,914,412;

(pyro) Glu-His-Trp-Ser-Tyr-D-Ser(t-But)-Leu-Arg-Pro-NHEt, U.S. Pat. 4,024,248, and Konig, W., et al, "Peptides: Chemistry, Structure and Biology — Proceedings of the Fourth American Peptide Symposium," R. Walter & J. Meienhofer, Eds, (1975), Ann Arbor Science Publications, p 883—888;

(pyro) Glu-His-Trp-Ser-Tyr-D-Ser(t-But)-Leu-Arg-Pro-AzaGly, Dutta, A. S., et al, *Biochem. Biophys. Res. Commum. 81,* 382 (1978);

(pyro) Glu-His-Trp-Ser-Tyr-D-His(Bzl)-Leu-Arg-Pro-NHEt, Vale, W. et al, "Peptides: Studies and Biological Function — Proceedings of the Sixth American Peptide Symposium," E. Gross & J. Meienhofer, Eds, Pierce Chem Co. (1979) pp 781—793;

(pyro) Glu-His-Trp-Ser-Tyr-D-pentamethyl-Phe-Leu-Arg-Pro-Gly$NH_2$, Coy, D. H., "Clinical Neurological Endocrinology — A Pathological Physical Approach," G. Tol Ed, (1979) p 83; and

(pyro) Glu-His-Trp-Ser-Tyr-3-(2-naphthyl)-D-alanyl-Leu-Arg-Pro-Gly$NH_2$, Nestor, J., Jr. et al, *J. Med. Chem., 25,* 795 (1982).

Preferred antagonist analogs of LHRH are the nona- and decapeptides from US—A—4 341 767 and EP—A—97 031.

Such antagonists include those of Formula (II):

A-B-C-D-Tyr-X-Y-Arg-Pro-E (II)
1 2 3 4 5 6 7 8 9 10

and the pharmaceutically acceptable salts thereof, wherein:

X is N,N'-guanido-disubstituted-D-argininyl or D-homoargininyl, D-argininyl, D-lysyl, or D-alanyl residue wherein one hydrogen on C—3 of the D-alanyl is replaced by:

a) a carbocyclic aryl-containing radical selected from phenyl substituted with three or more straight chain lower alkyl or alkoxy groups, trifluoromethyl, naphthyl, anthryl, fluorenyl, phenanthryl, biphenylyl and benzhydryl; or

b) a saturated carbocyclic radical selected from cyclohexyl substituted with three or more straight chain lower alkyl groups, perhydronaphthyl, perhydrobiphenylyl, perhydro-2,2-diphenylmethyl, and adamantyl; or

c) a heterocyclic aryl containing radical selected from radicals represented by the following structural formulas:

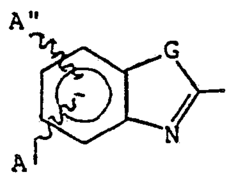
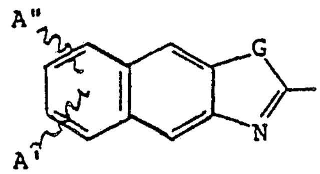
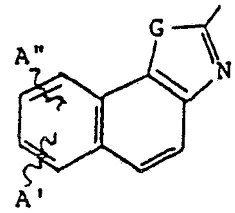
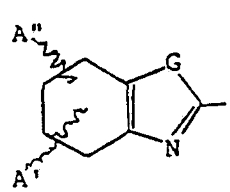
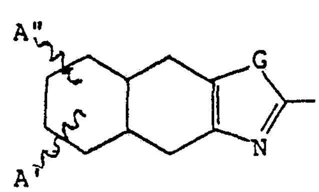
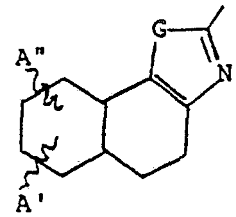

wherein A" and A' are independently selected from hydrogen, lower alkyl, chlorine, and bromine, and G is selected from oxygen, nitrogen, and sulfur;

A is an amino acyl residue selected from L-pyroglutamyl, D-pyroglutamyl, N-acyl-L-prolyl, N-acyl-D-prolyl, N-acyl-D-tryptophanyl, N-acyl-D-phenylalanyl, N-acyl-D-p-halophenylalanyl, N-acyl-D,L-seryl, N-acyl-D,L-threonyl, N-acyl-glycyl, N-acyl-D,L-alanyl, N-acyl-L-alkylprolyl, and N-acyl-X wherein X is as defined previously;

B is an amino acyl residue selected from D-phenylalanyl, D-p-halophenylalanyl, 2,2-diphenylglycyl, and X wherein X is as defined previously;

C is an amino acyl residue selected from L-tryptophanyl, D-tryptophanyl, D-phenylalanyl, D-phenylalanyl and X wherein X is as defined above;

D is an amino acyl residue selected from L-seryl, and D-alanyl;

Y is an amino acyl residue selected from L-leucyl, L-norleucyl and L-norvalyl;

E is D-alanyl, glycinamide or —NH—$R^1$, wherein $R^1$ is lower alkyl, cycloalkyl, fluoro lower alkyl or —NH—CO—NH—$R^2$ wherein $R^2$ is hydrogen or lower alkyl;

Those embodiments of Formula II most particularly preferred are those wherein the A group N-acyl is N-Ac, especially:

N-Ac-Pro-D-p-F-Phe-D-Nal(2)-Ser-Tyr-D-Nal(2)-Leu-Arg-Pro-Gly$NH_2$; and

N-Ac-Pro-D-p-Cl-Phe-D-Nal(2)-Ser-Tyr-D-Nal(2)-Leu-Arg-Pro-Gly$NH_2$.

Also particularly preferred are the following compounds which are disclosed in the noted patent and peridocal literature:

N-Ac-$\Delta^3$Pro-D-p-F-Phe-D-Nal(2)-Ser-Tyr-D-Nal(2)-Leu-Arg-Pro-Gly$NH_2$, Rivier, J., et al, "LHRH Peptides as Female and Male Contraceptives," G. I. Zatuchni, J. D. Snelton & J. J. Sciarra, Eds, Harper & Row, Philadelphia (1981), pp 13—23;

N-Ac-$\Delta^3$Pro-D-p-Cl-Phe-D-Trp-Ser-Tyr-D-Trp-N-Me-Leu-Arg-Pro-Gly$NH_2$, Rivier, J., et al, *Science,* 210, 93 (1980);

N-Ac-D-p-Cl-Phe-D-p-Cl-Phe-D-Trp-Ser-Tyr-D-Trp-Leu-Arg-Pro-D-Ala$NH_2$, Erchegyi, *Peptides, 2,*, 251 (1981);

N-Ac-D-p-Cl-Phe-D-p-Cl-Phe-D-Trp-Ser-Tyr-D-Arg-Leu-Arg-Pro-D-Ala$NH_2$, Coy, D. H., *Endocrinology, 110*, 1445 (1982); and

N-Ac-D-Nal(2)-D-p-F-Phe-D-Trp-Ser-Tyr-D-Arg-Leu-Arg-Pro-Gly$NH_2$, Rivier, J., et al, *Contraceptive Delivery Systems, 3,* 67 (1982).

Also useful are compounds of formula II but wherein A is N-acyl-$\Delta^3$-Pro, which can be prepared in analogous manner to the corresponding compounds of Rivier reference above.

Such antagonists also include those of formula

A-B-C-D-E-F-G-Arg-Pro-H (III)
1 2 3 4 5 6 7 8 9 10

and the pharmaceutically acceptable salts thereof, wherein:

A is an amino acyl residue selected from L-pyroglutamyl, D-pyroglutamyl, N-acyl-D,L-tryptophanyl, N-acyl-glycyl, N-Ac-D,L-$\Delta^{3,4}$-prolyl, N-Ac-D,L-prolyl, N-Ac-L-alkylprolyl, N-Ac-D,L-phenylalanyl, N-Ac-D,L-p-chlorophenylalanyl, N-Ac-D,L-seryl, N-Ac-D,L-threonyl, N-Ac-D,L-alanyl, 3-(1-naphthyl)-D,L-alanyl, 3-(2-naphthyl)-D,L-alanyl, 3-(2,4,6-trimethylphenyl)-D,L-alanyl, 3-(4-trifluoromethylphenyl)-D,L-alanyl, 3-(9-anthryl)-D,L-alanyl, 3-(2-fluoroenyl)-D,L-alanyl, and 3-(Het)-D,L-alanyl wherein Het is a heterocyclic aryl containing radical selected from

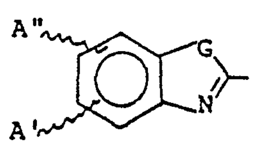

and

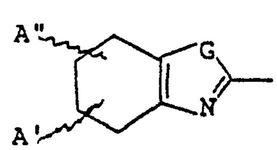

wherein A" and A' are independently selected from hydrogen, lower alkyl, chlorine and bromine, and G is selected from oxygen, nitrogen and sulfur;

B is an amino residue selected from D-phenylalanyl, D-p-Cl-phenylalanyl, D-p-F-phenylalanyl, D-p-nitrophenylalanyl, 3-(3,4,5-trimethoxyphenyl)-D-alanyl, 2,2-diphenylglycine, D-α-methyl-p-Cl-phenylalanine and 3-(2,4,6-trimethylphenyl)-D-alanyl;

C is an amino acyl residue selected from L-tryptophanyl, D-tryptophanyl, D-phenylalanyl, D-$Me_5$phenylalanyl, 3-(2-pyridyl)-D-alanyl, 3-(3-pyridyl)-D-alanyl, 3-(4-pyridyl)-D-alanyl, 3-(1-naphthyl)-D-alanyl, and 3-(2-naphthyl)-D-alanyl;

D is an amino acyl residue selected from L-seryl, and D-alanyl;

E is an amino acyl residue selected from L-phenylalanyl and L-tyrosyl;

F is an amino acyl selected from the radicals represented by the following structural formulas:

a)

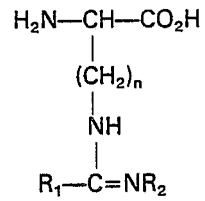

(II)

wherein

n is 1 to 5;

$R_1$ is alkyl of 1 to 12 carbon atoms, $-NRR_3$ wherein R is hydrogen or alkyl of 1 to 4 carbon atoms, $R_3$ is alkyl of 1 to 12 carbon atoms, cycloalkyl, phenyl, benzyl, $-(CH_2)_n$-morpholino or $-(CH_2)_nN(R_4)_2$ wherein n is 1 to 5 and $R_4$ is lower alkyl;

$R_2$ is hydrogen or $R_3$; or $R_1$ and $R_2$ comprise a ring represented by the following structural formulas:

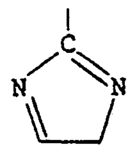
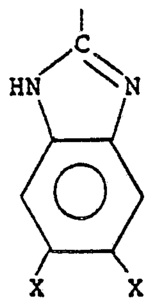
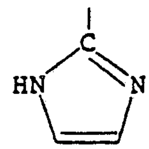
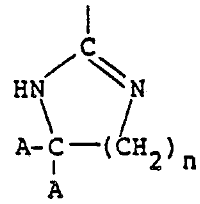

wherein n is 1 to 7; A is hydrogen, alkyl of 1 to 6 carbon atoms or cycloalkyl; and X is halo or A or

b)

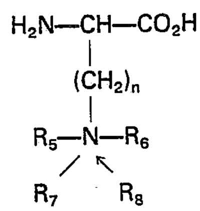

(III)

wherein $R_5$ is alkyl of 1 to 6 carbon atoms, benzyl, phenylethyl, cyclohexyl, cyclopentyl; and $R_6$, $R_7$ and $R_8$ are hydrogen or alkyl of 1 to 4 carbon atoms; and n is the integer 2—5; or

c) a substituent of the formula

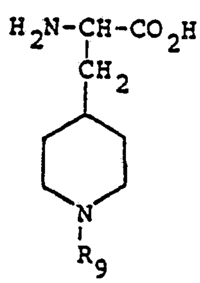

(IV)

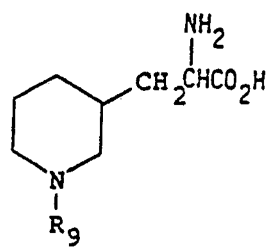

(V)

wherein $R_9$ is hydrogen, alkyl of 1 to 12 carbon atoms, phenyl or phenylloweralkyl;

G is an amino acyl residue selected from L-leucyl, L-norleucyl and L-norvalyl;

H is D-alaninamide, D-leucinamide, glycinamide or $-NHR_5$ wherein $R_5$ is lower alkyl, cycloalkyl, fluoro lower alkyl, or NHCONH—$R_{10}$ wherein $R_{10}$ is hydrogen or lower alkyl; and the pharmaceutically acceptable salts thereof.

Preferred examples of such antagonists include:

N-Ac-D-Nal(2)-D-pCl-Phe-D-Trp-Ser-Tyr-D-Deh-Leu-Arg-Pro-D-AlaNH$_2$;

N-Ac-D-Nal(2)-D-pCl-Phe-D-Trp-Ser-Tyr-D-Deh-Leu-Arg-Pro-GlyNH$_2$; and

N-Ac-D-Nal(2)-D-pF-Phe-D-Trp-Ser-Tyr-D-Deh-Leu-Arg-Pro-GlyNH$_2$ and their pharmaceutically

acceptable salts, and wherein D-Deh represents the residue of the amino acid N,N'-guanido-diethyl-D-homoarginine.

The amount of peptide present in the nasal formulation will often be between 0.005 and 5 milligrams per ml of solution, particularly with agonist peptides. Preferably, with agonist peptides there will be present an amount of 0.05 to 4 milligrams per mil, but most preferably the LHRH analog will be present in an amount of 0.1 to 2.0, or 0.1 ot 1.0,'milligrams per ml. With antagonist peptides, often higher concentrations will be used, such as 5 to 100 mg/ml, more often 5 to 20, for example 5—10 mg/ml.

The agent which is responsible for enhancing the absorption of LHRH compounds across the nasal membrane are bile acid surfactants, and their pharmaceutically acceptable salts.

These acids are, for example, glycocholic acid, cholic acid, taurocholic acid, cholanic acid, ethocholic acid, desoxycholic acid, chenodesoxychloic acid and dehydrocholic acid; also glycodeoxy-cholic acid. One or more acids or salts, but preferably a single pharmaceutically acceptable acid salt, is added to the aqueous solution.

The pharmaceutically acceptable surfactant salts will be any salt which retains the phenomena of enhanced peptide absorption, as well as the compounds' surfactant characteristics, and which are not deleterious to the subject or otherwise contraindicated. Such salts are for example those salts derived from inorganic bases which include sodium, potassium, lithium, ammonium, calcium, magnesium, ferrous, zinc, copper, manganous, aluminium, ferric and manganic salts. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triehylamine, tripropylamine, ethanolamine, 2-dimethylaminoethanol, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine and, polyamine resins. Particularly preferred organic non-toxic bases are isopropylamine, diethylamine, ethanolamine, tromethamine, dicyclohexylamine, choline and caffeine.

More preferably, the surfactant used in the practice of this invention will be an alkali metal salt of glycocholic acid, most preferably sodium glycocholate.

The amount of surfactant used for the practice of this invention will be some amount which increases the absorption of LHRH peptides over that of other surfactants which also may enhance peptide absorption to a certain degree. It has been found that such an an amount is often in the range between 0.2 and 15%, more often 0.2 to 5 percent by weight/volume of the solution. It is preferred that the surfactant be present in an amount between about 0.5 to 4 percent by weight/volume, conveniently about 1 percent by weight/volume, preferably about 2 percent by weight/volume.

The subject nasal formulations will be formulated in water but more preferably it will be formulated in a solution buffered to a pH of between about 3.0 and 8.0, most preferably pH 5.0—5.4, by means of some pharmaceutically acceptable buffer system. Any pharmaceutically acceptable buffering system capable of maintaining a pH in the denoted range may be used for the practice of this invention. A typical buffer will be, for example, an acetate buffer, a phosphate buffer, a citrate buffer or a succinate buffer. The buffer of choice here is an acetate buffer in a concentration of between 0.005 and 0.1 molar, most preferably 0.02 molar. Water or buffer solution is added in a quantity sufficient to make volume.

Other materials known as preservatives, salts to achieve the tonic value of tissue, or other additives indicted by known nasal formulation chemistry may be added to these formulations. Particularly advantageous other such materials include surfactants, suitably non-ionic surfactants such as the polysorbates, in concentrations suitably in the range 0.1 to 5, more suitably 0.25 to 2% weight volume.

It has been found that often to obtain enhanced solubility and stability, the molar ratio of bile acid to peptide is usefully ≥20:1, such as ≥25:1.

In one suitable embodiment, the present invention relates to a nasal spray composition having enhanced LHRH polypeptide absorption comprising 0.005 to 5 mg/ml of a nona- or decapeptide or its pharmaceutically acceptable salt having LHRH agonist or antagonist activity; 0.2 to 5% by weight/volume of a surfactant which is a bile acid or a pharmaceutically acceptable salt thereof; and a buffered aqueous solution in a quantity sufficient to make volume.

In a further suitable embodiment, this invention also relates to a method for preparing a medicament for enhancing the nasal uptake of a LHRH analog which method comprises adding 0.2 to 5 percent by weight/volume of a surfactant which is a bile acid or a pharmaceutically acceptable salt thereof to a nasal spray composition comprising 0.005 to 5 mg/ml of a nona- or decapeptide or its pharmaceutically acceptable salt having LHRH agonist or antagonist activity; and buffered aqueous solution in a quantity sufficient to make volume.

The process for preparing the nasal compositions of the invention comprises bringing into aqueous solution the peptide and the surfactant. As will be clear from the foregoing, this process may be carried out in known manner for preparing nasal aqueous compositions. Suitably, the peptide is first dissolved in buffer, and then added to the surfactant, the pH adjusted as required, and the volume made up to the desired level. Additional components can be added in at any convenient stage in the process.

The nasal compositions of this invention may be administered in conventional manner. For example,

sufficient composition to deliver an effective does of LHRH analog is administered to the nostrils, conveniently in a divided dose being administered to each nostril, suitably by means of a spray. Suitably a spray bottle with a metered dose (conveniently 100 µl per spray) spray attachment is used.

The invention is illustrated by the following nonlimiting examples.

Example 1

6.25 milligrams of (pyro)Glu - His - Trp - Ser - Tyr - 3 - (2 - naphthyl) - D - alanyl - Leu - Arg - Pro - Gly - $NH_2$ were dissolved in 5 ml of a 0.02 molar acetate buffer solution having a pH of about 5.2 in a volumetric flask. A hundred milligrams of sodium glycocholate were then dissolved in this solution which is brought almost to volume, the pH adjusted to 5.2 ± 0.2 and then a volume of buffer added in a quantity sufficient to make 10 ml.

Example 2

1 mg (pyro) Glu - His - Trp - Ser - Tyr - 3 - (2 - naphthyl) - D - alananyl - Leu - Arg - Pro - Gly - $NH_2$ was dissolved in 50 ml of a 0.02 molar acetate buffer solution having a pH of about 5.2, and was added to a solution containing 500 mg of sodium glycocholate, the pH adjusted to 5.2 ± 0.2, and then a volume of buffer added in a quantity sufficient to make 100 ml.

Example 3

2 mg of (pyro) Glu - His - Trp - Ser - Tyr - 3 - (2 - naphthyl) - D - analyl - Leu - Arg - Pro - Gly - $NH_2$ was dissolved in 5 ml of a 0.02 molar phosphate buffer having a pH of about 7.0, and was added to a solution containing 75 mg of sodium glycodeoxy-cholate, the pH adjusted to 7.0 ± 0.2, and then a volume of buffer added in a quantity sufficient to make 10 ml.

Exmaple 4

50 mg of N - Ac - D - Nal(2) - D - pCl - Phe - D - Trp - Ser - Tyr - D - Deh - Leu - Arg - Pro - D - $AlaNH_2$ was dissolved in 5 ml of a 0.02 molar acetate buffer solution having a pH of about 5.2, and was added to a solution containing 500 mg of sodium glycocholate, the pH adjusted to 5.2 ± 0.2, and then a volume of buffer added in quantity sufficient to make 10 ml.

Example 5

The procedure of Example 2 was repeated, but using 17.5 mg of peptide and 200 mg of sodium glycocholate.

Example 6

The procedure of Example 2 was repeated, but using 12.5 mg of peptide and 200 mg of sodium glycocholate.

Example 7

The procedure of Example 2 was repeated, but 0.5% (w/v) of Polysorbate® 20 (polyoxyethylene 20 sorbitan monolaurate) was also incorporated in the solution.

Example 8

The procedure of Example 2 was repeated, but using 10 mg ofpeptide and 150 mg of sodium glycocholate.

Note, in the Examples the peptides were used in the form of their acetate salts.

BIOLOGICAL DATA

The enhancement of absorption of (pyro)Glu - His - Trp - Ser - Tyr - 3 - (2 - naphthyl) - D - alanyl - Leu - Arg - Pro - Gly - $NH_2$ by sodium glycocholate is illustrated in the following Table I.

TABLE 1

| Formulation | Drug conc mg/ml | Avg dose per monkey μ G | Peak Plasma level ng/ml | Auc (8 h) |
|---|---|---|---|---|
| Standard Nasal | 0.625 | 133 | 0.23 (0.08) | 0.63 |
| +1% Sodium glycocholate | 0.625 | 120 | 11.1 (2.13) | 14.1 |
| Subcutaneous | | 5 | 1.9 (0.35) | 5.5 |

[Numbers in parenthesis are standard error. Standard nasal formulation is the peptide dissolved in acetate buffer, and at the same pH as the test formulation, but not including the sodium glycocholate.]

Further absorption tests were carried out in monkeys, as reported below in Table 2:

TABLE 2

| Formulation | Drug conc mg/ml | Avg dose per monkey μ G | Peak Plasma level ng/ml | Auc (8 h) |
|---|---|---|---|---|
| Standard nasal | 1.25 | 272 | 1.84 | 4.6 |
| +2% Sodium glycocholate | 1.25 | 243 | 26.4 | 38.3 |
| +1% Sodium glycocholate and 0.5% Polysorbate 20 | 0.625 | 123 | 5.5 | |

NASAL TOXICITY

The composition of Example 5 was administered to Beagle dogs intranasally at a dose of 0.4 ml per dog per day for 28 days. The nasal cavities of the dogs were examined, and no changes attributable to the composition were observed.

**Claims**

1. A nasal composition comprising a nona- or decapeptide or its pharmaceutically acceptable salt having LHRH agonist or antagonist activity, and a surfactant, which composition is characterized in that it is an aqueous formulation containing, as said surfactant, a bile acid or a pharmacuetically acceptable salt thereof.

2. A composition according to Claim 1 which is a nasal spray composition comprising 0.005 to 5 mg/ml of a nona- or decapeptide or its pharmaceutically acceptable salt having LHRH agonist or antagonist activity; 0.2 to 5 percent by weight/volumne of a surfactant which is a bile acid or a pharmaceutically acceptable salt thereof; and buffered aqueous solution in a quantity sufficient to make volume.

3. A composition according to Claim 22 wherein the LHRH compound is an agonist represented by the formula

(pyro)Glu-His-V-Ser-W-X-Y-Arg-Pro-Z (I)

and the pharmaceutically acceptable salts thereof wherein:

V is tryptophyl, phenylalanyl or 3-(1-naphthyl)-L-alanyl;

W is tyrosyl, phenylalanyl or 3-(1-pentafluorophenyl)-L-alanyl;

X is a D-amino acid residue

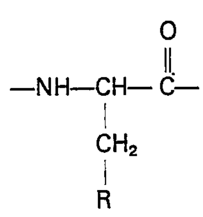

wherein R is

(a) a carbocyclic aryl-containig radical selected from naphthyl, anthryl, fluorenyl, phenanthryl, biphenylyl, benzhydryl and phenyl substituted with three or more straight chain lower alkyl groups; or

(b) a saturated carbocyclic radical selected from cyclohexyl substituted with three or more straight chain lower alkyl groups, perhydronaphthyl, perhydrobiphenylyl, perhydro-2,2-diphenylmethyl and adamantyl;

Y is leucyl, isoleucyl, nor-leucyl or N-methyl-leucyl;

Z is glycinamide or —NR—$R^1$, wherein $R^1$ is lower alkyl, cycloalkyl, fluoro lower alkyl or

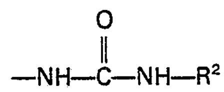

wherein $R^2$ is hydrogen or lower alkyl and the surfactant is an alkali metal salt of a bile acid.

4. A composition according to Claim 3 wherein said compound is (pyro)Glu - His - Trp - Ser - Tyr - 3 - (2 - naphthyl) - D - alanyl - Leu - Arg - Pro - Gly - $NH_2$ or a pharmaceutically acceptable salt thereof.

5. A composition according to Claim 4, wherein the peptide is present in an amount of 0.05 to 4 milligrams/ml and said surfactant is sodium glycocholate which is present in an amount of 0.5 to 4 percent weight/volume.

6. A composition according to Claim 1 wherein said compound is an antagonist having the formula

A-B-C-D-Tyr-X-Y-Arg-Pro-E (II)
1 2 3 4 5 6 7 8 9 10

and the pharmaceutically acceptable salts thereof, wherein:

X is N,N'-guanido-disubstituted-D-agininyl or D-homoargininyl, D-argininyl, D-lysyl, or D-alanyl residue wherein one hydrogen on C—3 of the D-alanyl is replaced by:

a) a carbocyclic aryl-containing radical selected from phenyl substituted with three or more straight chain lower alkyl or alkoxy groups, trifluoromethyl, naphthyl, anthryl, fluorenyl, phenanthryl, biphenylyl and benzhydryl; or

b) a saturated carbocyclic radical selected from cyclohexyl substituted with three or more straight chain lower alkyl groups, perhydronaphthyl, perhydrobiphenyl, perhydro-2,2-diphenylmethyl, and adamantyl; or

c) a heterocyclic aryl containing radical selected from radicals represented by the following structural formulas:

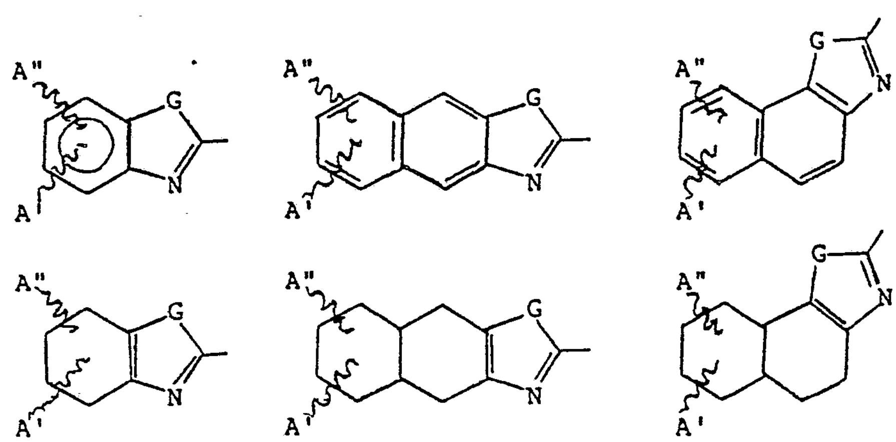

wherein A″ and A′ are independently selected from the group consisting of hydrogen, lower alkyl, chlorine, and bromine, and G is selected from oxygen, nitrogen, and sulfur;

A is an amino acyl residue selected from L-pyroglutamyl, D-pyroglutamyl, N-acyl-L-prolyl, N-acyl-D-prolyl, N-acyl-D-tryptophanyl, N-acyl-D-phenylalanyl, N-acyl-D-p-halophenylalanyl, N-acyl-D,L-seryl, N-acyl-D,L-threonyl, N-acyl-glycyl, N-acyl-D,L-alanyl, N-acyl-L-alkylprolyl, and N-acyl-X wherein X is as defined previously;

B is an amino acyl residue selected from D-phenylalanyl, D-p-halophenylalanyl, 2,2-diphenylglycyl, and X wherein X is as defined previously;

C is an amino acyl residue selected from L-tryptophanyl, D-tryptophanyl, D-phenylalanyl, D-phenylalanyl and X wherein X is as defined above;

D is an amino acyl residue selected from L-seryl, and D-alanyl;

Y is an amino acyl residue selected from L-leucyl, L-norleucyl and L-norvalyl;

E is D-alanyl, glycinamide or —NH—$R^1$, wherein $R^1$ is lower alkyl, cycloalkyl, fluoro lower alkyl or —NH—CO—NH—$R^2$ wherein $R^2$ is hydrogen or lower alkyl.

7. A composition according to claim 1, wherein said compound is an antagonist having the formula

A-B-C-D-E-F-G-Arg-Pro-H (III)
1 2 3 4 5 6 7 8 9 10

and the pharmaceutically acceptable salts thereof, wherein:

A is an amino acyl residue selected from L-pyroglutamyl, D-pyroglutamyl, N-acyl-D,L-tryptophanyl, N-acyl-glycyl, N-Ac-D,L-$\Delta^{3,4}$-prolyl, N-Ac-D,L-prolyl, N-Ac-L-alkylprolyl, N-Ac-D,L-phenylalanyl, N-Ac-D,L-p-chlorophenylalanyl, N-Ac-D,L-seryl, N-Ac-D,L-thereonyl, N-Ac-D,L-alanyl, 3-(1-naphthyl)-D,L-alanyl, 3-(2-naphthyl)-D,L-alanyl, 3-(2,4,6-trimethylphenyl)-D,L-alanyl, 3-(4-trifluoromethylphenyl)-D,L-alanyl, 3-(9-anthryl)-D,L-alanyl, 3-(2-fluorenyl)-D,L-alany, and 3-(Het)-D,L-alanyl wherein Het is a heterocyclic aryl containing radical selected from

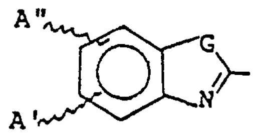
and
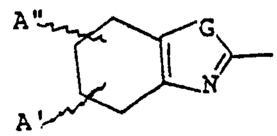

wherein A″ and A′ are independently selected from hydrogen, lower alkyl, chlorine and bromine, and G is selected from oxygen, nitrogen and sulfur;

B is an amino acyl residue selected from D-phenylalanyl, D-p-Cl-phenylalanyl, D-p-F-phenylalanyl, D-p-nitrophenylalanyl, 3-(3,4,5-trimethoxyphenyl)-D-alanyl, 2,2-diphenylglycine, D-α-methyl-p-Cl-phenylalanine and 3-(2,4,6-trimethylphenyl)-D-alanyl;

C is an amino acyl residue selected from L-tryptophanyl, D-tryptophanyl, D-phenylalanyl, D-$Me_5$-phenylalanyl, 3-(2-pyridyl)-D-alanyl, 3-(3-pyridyl)-D-alanyl, 3-(4-pyridyl)-D-alanyl, 3-(1-naphthyl)-D-alanyl, and 3-(2-naphthyl)-D-alanyl;

D is an amino acyl residue selected from L-seryl, and D-alanyl;

E is an amino acyl residue selected from the group consisting of L-phenylalanyl and L-tyrosyl;

F is an amino acyl selected from the radicals represented by the following structural formulas:

a)

$H_2N—CH—CO_2H$ (II)
|
$(CH_2)_n$
|
NH
|
$R_1—C{=}NR_2$

wherein

n is 1 to 5;

$R_1$ is alkyl of 1 to 12 carbon atoms, $—NRR_3$ wherein R is hydrogen or alkyl of 1 to 4 carbon atoms, $R_3$ is alkyl of 1 to 12 carbon atoms, cycloalkyl, phenyl, benzyl, $—(CH_2)_n$-morpholino or $—(CH_2)_nN(R_4)_2$ wherein n is 1 to 5 and $R_4$ is lower alkyl;

$R_2$ is hydrogen or $R_3$; or $R_1$ and $R_2$ comprise a ring represented by the following structural formulas:

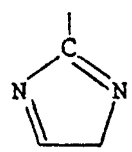
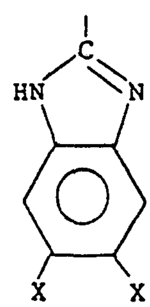
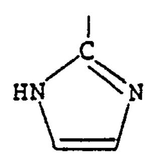
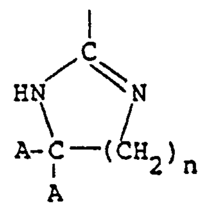

wherein n is 1 to 7; A is hydrogen, alkyl of 1 to 6 carbon atoms or cycloalkyl; and X is halo or A or

b)

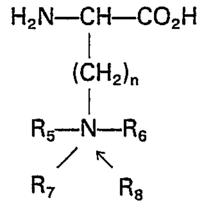

(III)

wherein $R_5$ is alkyl of 1 to 6 carbon atoms, benzyl, phenylethyl, cyclohexyl, cyclopentyl; and $R_6$, $R_7$ and $R_8$ are hydrogen or alkyl of 1 to 4 carbon atoms; and n is the integer 2—5; or

c) a substituent of the formula

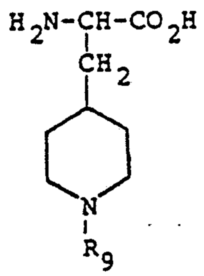

(IV)

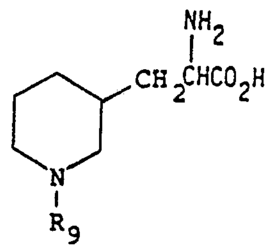

(V)

wherein $R_9$ is hydrogen, alkyl of 1 to 12 carbon atoms, phenyl or phenylloweralkyl;

G is an amino acyl residue selected from L-leucyl, L-norleucyl and L-norvalyl;

H is D-alaninamide, D-leucinamide, glycinamide or —$NHR_5$ wherein $R_5$ is lower alkyl, cycloalkyl, fluoro lower alkyl, or NHCONH—$R_{10}$ wherein $R_{10}$ is hydrogen or lower alkyl; and the pharmaceutically acceptable salts thereof.

8. A composition according to Claim 7, wherein the compound is N -Ac -D -Nal(2) -D -pCl -Phe -D -Trp -Ser -Tyr -D -Deh -Leu -Arg -Pro -D -$AlaNH_2$ or a pharmaceutically acceptable salt thereof.

9. A composition according to any one of the preceding claims, also including an additional surfactant.

10. A composition according to any one of the preceding claims, wherein the molar ratio of bile acid to peptide is ≥20:1.

11. A method of preparing a medicament for enhancing the nasal uptake of a LHRH analog, which comprises adding a surfactant which is a bile acid or a pharmaceutically acceptable salt thereof to a nasal composition comprising a nona- or decapeptide or a pharmaceutically acceptable salt thereof having LHRH agonist or antagonist activity formulated in an aqueous solution.

12. A method according to Claim 11 which comprises adding 0.2 to 5 percent by weight/volume of a surfactant which is a bile acid or a pharmaceutically acceptable salt thereof to a nasal spray composition comprising 0.005 to 5 mg/ml of a nona- or decapeptide or its pharmaceutically acceptable salt having LHRH agonist or antagonist activity; and buffered aqueous solution in a quantity sufficient to make volume.

**Patentansprüche**

1. Nasale Zusammensetzung, umfassend ein Nona oder Decapeptid oder dessen pharmazeutisch verträgliches Salz mit LHRH-Agonist- oder Antagonist-Aktivität und ein Tensid, dadurch gekennzeichnet, daß die Zusammensetzung eine wässrige Formulierung ist, die als Tensid eine Gallensäure oder ein pharmazeutisch verträgliches Salz davon enthält.

2. Zusammensetzung gemäß Anspruch 1, nämlich eine Nasen-spray-Zusammensetzung umfassend 0,005 bis 5 mg/ml eines Nona- oder Decapeptids oder dessen pharmazeutisch verträglichen Salzes mit LHRH-Agonist- oder Antagonist-Aktivität; 0,2 bis 5% Gew./Volumen eines Tensids, welches eine Gallensäure oder ein pharmazeutisch verträgliches Salz davon ist; und eine gepufferte wässerige Lösung in einer Menge die ausreicht, um ein gewisses Volumen zu schaffen.

3. Zusammensetzung gemäß Anspruch 2, in welcher die LHRH-Verbindung ein Agonist, dargestellt durch die Formel

(pyro)Glu-His-V-Ser-W-X-Y-Arg-Pro-Z (I)

und dessen pharmazeutisch annehmbare Salze ist, wobei:

V für Tryptophyl, Phenylalanyl oder 3-(1-Naphthyl)-L-alanyl steht;

W Tyrosyl, Phenylalanyl oder 3-(1-Pentafluorhenyl)-L-alanyl darstellt;

X ein D-Aminosäurerest

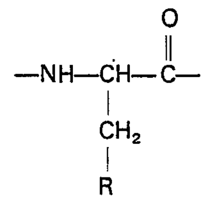

ist, in welchem R

(a) eine carbocyclisches Aryl enthaltende Gruppe, aus gewählt aus Naphthyl, Anthryl, Flurorenyl, Phenanthryl, Biphenylyl, Benzhydryl und Phenyl, substituiert mit 3 oder mehr geradkettigen Niederalkylgruppen; oder

(b) eine gesättigte carbocyclische Gruppe, ausgewählt aus Cyclohexyl, substituiert mit drei oder mehr geradkettigen Niederalkylgruppen, Perhydronaphthyl, Perhydrobiphenylyl, Perhydro-2,2-diphenylmethyl und Adamantyl darstellt;

Y für Leucyl, Isoleucyl, Norleucyl oder N-Methylleucyl steht;

Z Glycinamid oder —NR—$R^1$ ist, in welchem $R^1$ Niederalkyl, Cycloalkyl, Fluor-Niederalkyl oder

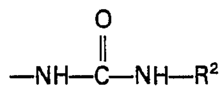

ist, wobei $R^2$ für Waswerstoff oder Niederalkyl steht; und das Tensid ein Alkalimetallsalz einer Gallensäure ist.

4. Zusammensetzung gemäß Anspruch 3, in welcher die Verbindung (pyro)Glu - His - Tryp - Ser - Tyr - 3 - (2 - naphthyl) - D - alanyl - Leu - Arg - Pro - Gly - $NH_2$ oder ein pharmazeutisch verträgliches Salz davon ist.

5. Zusammensetzung gemäß Anspruch 4, in welcher das Peptid in einer Menge von 0,05 bis 4 mg/ml anwesend und das Tensid Natrium-Glycocholate ist, das in einer Menge von 0,5 bis 4% Gew/Volumen vorhanden ist.

6. Zusammensetzung gemäß Anspruch 1, in welcher die Verbindung ein Antagonist mit der Formel

A-B-C-D-Tyr-X-Y-Arg-Pro-E (II)
1 2 3 4 5 6 7 8 9 10

und pharmazeutisch verträgliche Salze davon ist, wobei: X darstellt einen N,N'-Guanido-disubstituiertes D-Argininyl- oder D-Homoargininyl-, D-Argininyl-, D-Lysyl- oder D-Alanyl-Rest, in welchem ein Wasserstoff am C—3 des D-Alanyl ersetzt ist durch:

a) eine carbocyclisches Aryl enthaltende Gruppe, ausgewählt aus Phenyl, substituiert mit drei oder mehr geradkettigen Niederalkyl- oder Alkoxygruppen, Trifluormethyl, Naphthyl, Anthryl, Fluorenyl, Phenanthryl, Biphenylyl und Benzhydryl; oder

b) eine gesättigte carbocyclische Gruppe ausgewählt aus Cyclohexyl, substituiert mit drei oder mehr geradkettigen Niederalkylgruppen, Perhydronaphthyl, Perhydrobiphenylyl, Perhydro-2,2-diphenylmethyl und Adamantyl; oder

c) eine heterocyclisches Aryl enthaltende Gruppe ausgewählt aus Gruppen, die durch die folgenden Strukturformeln dargestellt werden:

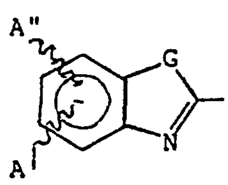
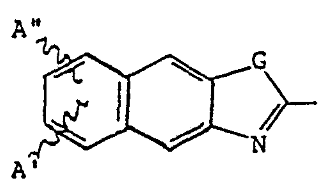
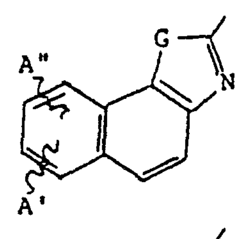
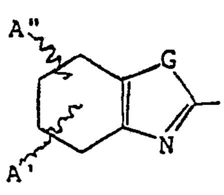
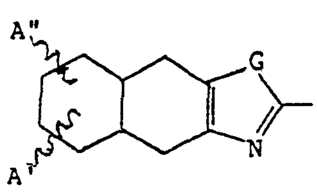
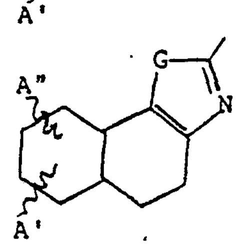

in welchen A" und A' unabhängig voneinander aus der Gruppe bestehend aus Wasserstoff, Niederalkyl, Chlor und Bro, und G aus Sauerstoff, Stickstoff und Schwefel augewählt werden;

A ein Aminoacyl-Rest, ausgewählt aus L-Pyroglutamyl, D-Pyroglutamyl, N-Acyl-L-prolyl, N-Acyl-D-prolyl, N-Acyl-D-tryptophanyl, N-Acyl-D-phenylalanyl, N-Acyl-D-p-halopenylalanyl, N-Acyl-D,L-seryl, N-Acyl-D,L-threonyl, N-Acyl-glycyl, N-Acyl-D,L-alanyl, N-Acyl-alkylprolyl und N-Acyl-X, wobei X wie vorstehend definiert ist, ist;

B für einen Aminoacyl-Rest, ausgewählt aus D-Phenylalanyl, D-p-Halophenylalanyl, 2,2-Diphenylglycyl und

X, wobei X wie vorstehend definiert ist, steht;

C einen Aminoacyl-Rest, ausgewählt aus L-Tryptophanyl,

D-Tryptophanyl, D-Phenylalanyl und X, wobei X wie vorstehend definiert ist, bedeutet;

D ein Aminoacyl-Rest, ausgewählt aus L-Seryl und D-Alanyl, ist;

Y ein Aminoacyl-Rest, ausgewählt aus L-Leucyl, L-Norleucyl und L-Norvalyl, ist;

E D-Alanyl, Glycinamid oder- NH—$R^1$ bedeutet, wobei $R^1$ Niederalkyl, Cycloalkyl, Fluor-Niederalkyl oder —NH—CO—NH—$R^2$ ist, wobei $R^2$ für Wasserstoff oder Niederalkyl steht.

7. Zusammensetzung gemäß Anspruch 1, in welcher die Verbindung ein Antagonist mit der Formel

A-B-C-D-E-F-G-Arg-Pro-H (III)
1 2 3 4 5 6 7 8 9 10

und deren pharmazeutisch verträgliche Salze ist, wobei: A eien Amioacyl-Rest, ausgewählt aus L-Pyroglutamyl, D-Pyroglutamyl, N-Acyl-D,L-tryptophanyl, N-Acyl-Glycyl, N-Ac-D,L-$\Delta^{3,4}$-Prolyl, N-Ac-D,L-Prolyl, N-Ac-L-Alkylprolyl, N-Ac-D,L-Phenylalanyl, N-Ac-D,L-p-Chlorphenylalanyl, N-Ac-D,L-Seryl, N-Ac-D,L-Threonyl, N-Ac-D,L-Alanyl, 3-(1-Naphthyl)-D,L-alanyl, 3-(2-Naphthyl)-D,L-alanyl, 3-(2,4,6-Trimethylphenyl)-D,L-alanyl, 3-(4-Trifluormethylphenyl)-D,L-alanyl, 3-(9-Anthryl)-D,L-alanyl, 3-(2-flurenyl)-D,L-alanyl, und 3-(Het)-D,L-alanyl, darstellt, wobei Het eine heterocyclisches Aryl enthaltende Gruppe, ausgewählt aus

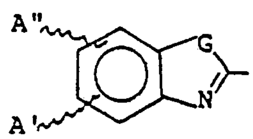

und

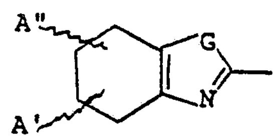

ist,
in welchen A″ und A′ unabhängig voneinander ausgewählt sind aus Wasserstoff, Niederalkyl, Chlor und Brom und G ausgewählt ist aus Sauerstoff, Stickstoff und Schwefel;

B einen Aminoacyl-Rest, ausgewählt aus D-Phenylalanyl, D-p-Cl-Phenylalanyl, D-p-F-Phenylalanyl, D-p-Nitrophenylalanyl, 3-(3,4,5-Trimethoxyphenyl)-D-alanyl, 2,2-Diphenylglycin, D-α-Methyl-p-Cl-phenylalanin und 3-(2,4,6-Trimethylphenyl)-D-alanyl, darstellt;

C für einen Aminoacyl-Rest, ausgewählt aus L-Tryptophanyl, D-Tryptophanyl, D-Phenylalanyl, D-Me-Phenylalanyl, 3-(2-Pyridyl)-D-alanyl, 3-(3-Pyridyl)-D-alanyl, 3-(4-Pyridyl)-D-alanyl, 3-(1-Naphthyl)-D-alanyl und 3-(2-Naphthyl)-D-alanyl, steht;

D ein Aminoacyl-Rest, ausgewählt aus L-Seryl und D-Alanyl, ist;

E einen Aminoacyl-Rest, ausgewählt aus der Gruppe bestehend aus L-Phenylalanyl und L-Tyrosyl, repräsentiert;

F ein Aminoacyl-Rest, ausgewählt aus Gruppen, die durch die folgenden Strukturformeln repräsentiert werden, ist:

a)

$H_2N$—CH—$CO_2H$
|
$(CH_2)_n$ (II)
|
NH
|
$R_1$—C=$NR_2$

in welcher

n 1 bis 5 ist;

$R_1$ für Alkyl mit 1 bis 12 Kohlenstoffatomen und —$NRR_3$ steht, wobei R Wasserstoff oder Alkyl mit 1 bis 4 Kohlenstoffatomen, $R_3$ Alkyl mit 1 bis 12 Kohlenstoffatomen, Cycloalkyl, Phenyl, Benzyl, —$(CH_2)_n$-Morpholino oder -$(CH_2)_nN(R_4)_2$ ist, wobei n für 1 bis 5 und $R_4$ für Niederalkyl steht;

$R_2$ Wasserstoff oder $R_3$ darstellt; oder $R_1$ und $R_2$ einen durch die folgenden Strukturformeln dargestellten Ring umfassen:

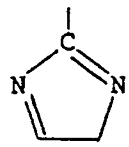
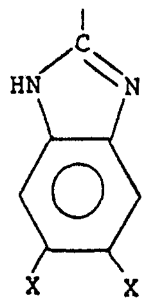
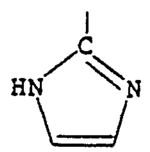
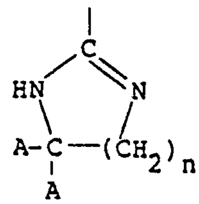

in welchen n 1 bis 7 ist; A für Wassrstoff, Alkyl mit 1 bis 6 Kohlenstoffatomen oder Cycloalkyl steht; und X steht für Halo oder A oder

b)

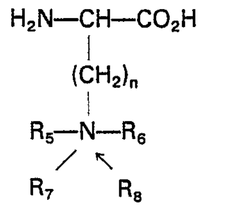

(III)

wobei $R_5$ Alkyl mit 1 bis 6 Kohlenstoffatomen, Benzyl, Phenylethyl, Cyclohexyl, Cyclopentyl ist; und $R_6$, $R_7$ und $R_8$ Wasserstoff oder Alkyl mit 1 bis 4 Kohlenstoffatomen bedeuten; und n die ganzen Zahlen 2 bis 5 repräsentiert; oder

c) einen Substituenten der Formel

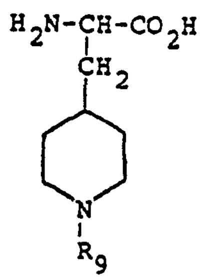

(IV)

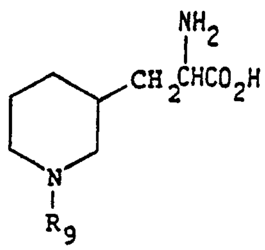

(V)

in welchen $R_9$ Wasserstoff, Alkyl mit 1 bis 12 Kohlenstoffatomen, Phenyl oder Phenyl-Niederalkyl bedeutet;

G ein Aminoacyl-Rest, ausgewählt aus L-Leucyl, L-Norleucyl und L-Norvalyl, ist;

H D-Alaninamid, D-Leucinamid, Glycinamid oder —$NHR_5$ bedeutet, wobei $R_5$ Niederalkyl, Cycloalkyl, Fluor-Niederalkyl oder NHCONH-$R_{10}$ ist, wobei $R_{10}$ für Wasserstoff oder Niederalkyl steht; und deren pharmazeutisch verträgliche Salze.

8. Zusammensetzung gemäß Anspruch 7, in welcher die Verbindung N - Ac - D - Nal(2) - D - pCl - Phe - D - Trp - Ser - Tyr - D - Deh - Leu - Arg - Pro - D - $AlaNH_2$ oder ein pharmazeutisch verträgliches Salz davon ist.

9. Verbindung gemäß einem der vorangehenden Ansprüche, die auch ein zusätzliches Tensid beinhaltet.

10. Zusammensetzung gemäß einem der vorangehenden Ansprüche, in welcher das Molverhältnis von Gallensäure zu Peptid $\geq 20{:}1$ beträgt.

11. Verfahren zuer Herstellung eines Medikaments zur Verbesserung der nasalen Aufnahme eines LHRH-Analogen, umfassend die Zugabe eines Tensids, welches eine Gallensäure oder ein pharmazeutisch verträgliches Salz davon ist, zu einer nasalen Zusammensetzung, die ein Nona- oder Decapeptid oder ein pharmazeutisch verträgliches Salz davon mit LHRH-Agonist- oder Antagonist-Aktivität, formuliert in einer wässrigen Lösung, umfaßt.

12. Verfahren gemäß Anspruch 11, welches umfaßt die Zugabe von 0,2 bis 5% Gew./Volumen eines Tensids, welches eine Gallensäure oder ein pharmazeutisch verträgliches Salz davon ist, zu einer Nasenspray-Zusammensetzung, die 0,005 bis 5 mg/ml eines Nona- oder Decapeptids oder dessen pharmazeutisch verträglichen Salzes mit LHRH-Agonist- oder Antagonist-Aktivatät; und eine gepufferte wässrige Lösung in einer zur Schaffung eines gewissen Volumens ausreichenden Menge umfaßt.

**Revendications**

1. Composition nasale comprenant un nonapeptide ou décapeptide ou son sel pharmaceutiquement acceptable doué d'activité agoniste ou antagoniste de l'hormone LHRH, et un surfactant, composition caractérisée en ce qu'elle est une formulation aqueuse contenant, comme surfactant, un acide biliaire ou un sel pharmaceutiquement acceptable de cet acide.

2. Composition suivant la revendication 1, qui est une composition nasale pulvérisable comprenant 0,005 à 5 mg/ml d'un nonapeptide ou décapeptide ou son sel pharmaceutiquement acceptable doué d'activité agoniste ou antagoniste de l'hormone LHRH; 0,2 à 5% en poids/volume d'un surfactant qui est un acide biliaire ou un sel pharmaceutiquement acceptable de cet acide; et une solution aqueuse tamponnée en une quantité suffisante pour compléter le volume.

3. Composition suivant la revendication 2, dans laquelle le composé du type LHRH est un agoniste représenté par la formule

(pyro)Glu-His-V-Ser-W-X-Y-Arg-Pro-Z (I)

et les sels pharmaceutiquement acceptables correspondants, formule dans laquelle:

V est un groupe tryptophyle, phénylalanyle ou 3-(1-naphtyl)-L-alanyle;

W est un groupe tyrosyle, phénylalanyle ou 3-(1-pentafluorophényl)-L-alanyle;

X est un résidu de D-amino-acide

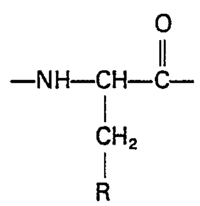

où R représente

(a) un radical carbocyclique contenant un groupe aryle choisi entre les radicaux naphtyle, anthryle, fluorényle, phénanthryle, biphénylyle, benzhydryle et phényle substitués avec trois ou plus de trois groupes alkyle inférieurs à chaîne droite; ou

(b) un radical carbocyclique saturé choisi entre un radical cyclohexyle substitué avec trois ou plus de trois groupes alkyle inférieurs à chaîne droite, perhydronaphtyle, perhydrobiphénylyle, perhydro-2,2-diphénylméthyle et adamantyle;

Y est un groupe leucyle, isoleucyle, norleucyle ou N-méthyl-leucyle;

Z est le glycinamide ou un groupe —NR—$R^1$, dans lequel $R^1$ est un radical alkyle inférieur, cycloalkyle, fluoralkyle inférieur ou un radical de formule

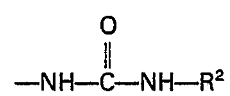

dans laquelle $R^2$ est l'hydrogène ou un groupe alkyle inférieur et le surfactant est un sel de métal alcalin d'un acide biliaire.

4. Composition suivant la revendication 3, dans laquelle ledit composé est le (pyro)Glu - His - Trp - Ser - Tyr - 3 - (2 - naphtyl) - D - alanyl - Leu - Arg - Pro - Gly - $NH_2$ ou un sel pharmaceutiquement acceptable de ce composé.

5. Composition suivant la revendication 4, dans laquelle le peptide est présent en une quantité de 0,05 à 4 mg/ml et ledit surfactant est le glycocholate de sodium qui est présent en une quantité de 0,5 à 4% en poids/volume.

6. Composition suivant la revendication 1, dans lequelle ledit composé est un antagoniste de formule

A-B-C-D-Tyr-X-Y-Arg-Pro-E (II)
1 2 3 4 5 6 7 8 9 10

et ses sels pharmaceutiquement acceptables, formule dans laquelle:

X est un groupe N,N'-guanido-disubstitué-D-argininyle ou D-homo-argininyle, D-argininyle, D-lysyle, ou un résidu D-alanyle dont un atome d'hydrogène sur l'atome C—3 est remplacé par:

a) un radical carbocyclique contenant un groupe aryle, choisi entre un radical phényle substitué avec trois ou plus de trois groupes alkyle ou alkoxy inférieurs à chaîne droite trifluorométhyle, naphtyle, anthryle, fluorényle, phénanthryle, biphénylyle et benzhydryle; ou bien

b) un radical carbocyclique saturé choisi entre un radical cyclohexyle substitué avec trois ou plus de trois groupes alkyle inférieurs à chaîne droite, perhydronaphtyle, perhydrobiphénylyle, perhydro-2,2-diphényléthyle et adamantyle; ou bien

c) un radical hétérocyclique contenant un groupe aryle, choisi entre des radicaux représentés par les formules structurales suivantes:

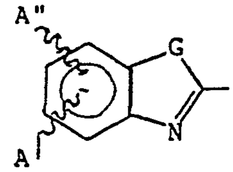
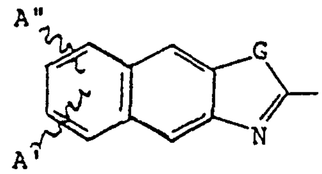
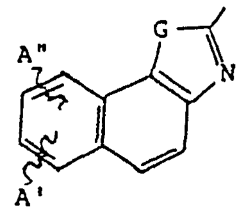
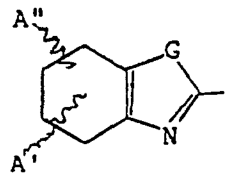
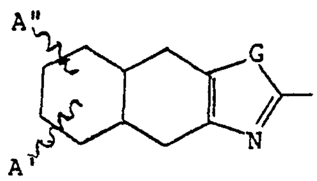
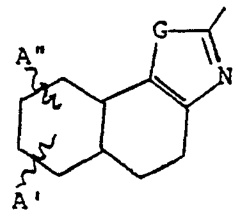

dans lesquelles A″ et A′ sont choisis, indépendamment, dans le groupe comprenant l'hydrogène, un radical alkyle inférieur, le chlore et le brome et G est choisi entre l'oxygène, l'azote et le soufre;

A est un résidu amino-acyle choisi entre les résidus L-pyroglutamyle, D-pyroglutamyle, N-acyl-L-prolyle, N-acyl-D-prolyle, N-acyl-D-tryptophanyle, N-acyl-D-phénylalanyle, N-acyl-D-p-halogénophénylalanyle, N-acyl-D,L-séryle, N-acyl-D,L-thréonyle, N-acyl-glycyle, N-acyl-D,L-alanyle, N-acyl-L-alkylprolyle, et N-acyl- X où X a la définition donnée ci-dessus;

B est un résidu amino-cycle choisi entre D-phénylalanyle, D-p-halogénophénylalanyle, 2,2-diphénylglycyle et X, ce dernier ayant la définition donnée ci-dessus;

C est un résidu amino-acyle choisi entre L-tryptophanyle, D-tryptophanyle, D-phénylalanyle, D-phénylalanyle et X, ce dernier ayant la définition donnée ci-dessus;

D est un résidu amino-acyle choisi entre L-séryle et D-alanyle;

Y est un résidu amino-acyle choisi entre L-leucyle, L-norleucyle et L-norvalyle;

E est un groupe D-alanyle, glycinamide ou —NH—$R^1$, où $R^1$ est un radical alkyle inférieur, cycloalkyle, fluoralkyle inférieur ou —NH—CO—NH—$R^2$, $R^2$ étant un atome d'hydrogène ou un groupe alkyle inférieur.

7. Composition suivant la revendication 1, dans laquelle ledit composé est un antagoniste répondant à la formule

A-B-C-D-E-F-G-Arg-Pro-H (III)
1 2 3 4 5 6 7 8 9 10

et ses sels pharmaceutiquement acceptables, formule dans laquelle:

A est un résidu amino-acyle choisi entre L-pyroglutamyle, D-pyroglutamyle, N-acyl-D,L-tryptophanyle, N-acyl-glycyle, N-Ac-D,L-$\Delta^{3,4}$-prolyle, N-Ac-D,L-prolyle, N-Ac-L-alkylprolyle, N-Ac-D,L-phénylalanyle, N-Ac-D,L-p-chlorophénylalanyle, N-Ac-D,L-séryle, N-Ac-D,L-thréonyle, N-Ac-D,L-alanyle, 3-(1-naphtyl)-D,L-alanyle, 3-(2-naphtyl)-D,L-alanyle, 3-(2,4,6-triméthylphényl)-D,L-alanyle, 3-(4-trifluorométhylphényl)-D,L-alanyle, 3-(9-anthryl)-D,L-alanyle, 3-(2-fluorényl)-D,L-alanyle et 3-(Het)-D,L-alanyle où Het est un radical hétérocyclique contenant un groupe aryle, choisi entre

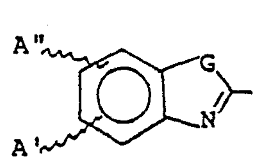 et 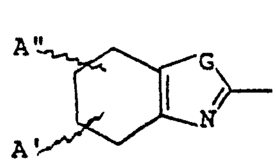

où A″ et A′ sont choisis, indépendamment, entre l'hydrogène, un radical alkyle inférieur, le chlore et le brome et G est choisi entre l'oxygène, l'azote et le soufre;

B est un résidu amino-acyle choisi entre D-phénylalanyle, D-p-Cl-phénylalanyle, D-p-F-phénylalanyle, D-p-nitrophénylalanyle, 3-(3,4,5-triméthoxyphényl)-D-alanyle, 2,2-diphénylglycine, D-α-méthyl-p-Cl-phénylalanine et 3-(2,4,6-triméthylphényl)-D-alanyle;

C est un résidu amino-acyle choisi entre L-tryptophanyle, D-tryptophanyle, D-phénylalanyle, D-$Me_5$phénylalanyle, 3-(2-pyridyl)-D-alanyle, 3-(3-pyridyl)-D-alanyle, 3-(4-pyridyl)-D-alanyle, 3-(1-naphtyl)-D-alanyle et 3-(2-naphtyl)-D-alanyle;

D est un résidu amino-acyle choisi entre L-séryle et D-alanyle;

E est un résidu amino-acyle choisi dans le groupe comprenant les résidus L-phénylalanyle et L-tyrosyle;

F est un résidu amino-acyle choisi entre les radicaux représentés par les formules structurales suivantes:

a)

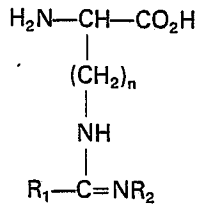

(II)

où

*n* a une valeur de 1 à 5;

$R_1$ est un groupe alkyle de 1 à 12 atomes de carbone, un groupe $—NRR_3$ dans lequel R est l'hydrogène ou un radical alkyle de 1 à 4 atomes de carbone, $R_3$ est un radical alkyle de 1 à 12 atomes de carbone, cycloalkyle, phényle, benzyle, $—(CH_2)_n$-morpholinio ou $—(CH_2)_nN(R_4)_2$ où *n* a une valeur de 1 à 5 et $R_4$ est un groupe alkyle inférieur;

$R_2$ est l'hydrogène ou $R_3$; ou bien $R_1$ et $R_2$ comprennent un noyau représenté par les formules structurales suivantes:

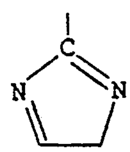

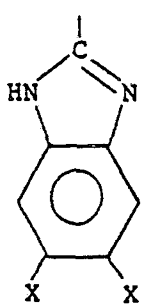

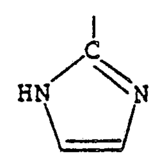

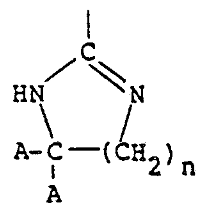

dans lesquelles *n* a une valeur de 1 à 7; A est l'hydrogène, un groupe alkyle de 1 à 6 atomes de carbone ou cycloalkyle; et X est un radical halogéno ou A, ou bien

b)

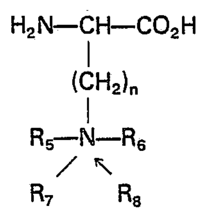

(III)

dans laquelle $R_5$ est un groupe alkyle de 1 à 6 atomes de carbone, benzyle, phényléthyle, cyclohexyle, cyclopentyle et $R_6$, $R_7$ et $R_8$ représentent l'hydrogène ou un groupe alkyle ayant 1 à 4 atomes de carbone; et *n* est un nombre entier de 2 à 5; ou bien

c) un substituant de formule

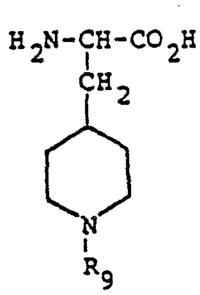

(IV)

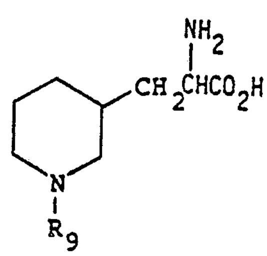

(V)

où $R_9$ est l'hydrogène, un groupe alkyle de 1 à 12 atomes de carbone, phényle ou phényl-(alkyle inférieur);

G est un résidu amino-acyle choisi entre L-leucyle, L-norleucyle et L-norvalyle;

H est un groupe D-alaninamide, D-leucinamide, glycinamide ou $—NHR_5$ dans lequel $R_5$ est un radical alkyle inférieur, cycloalkyle, fluoralkyle inférieur ou $NHCONH—R_{10}$ dans lequel $R_{10}$ est l'hydrogène ou un radical alkyle inférieur; et ses sels pharmaceutiquement acceptables.

8. Composition suivant la revendication 7, dans lequelle le composé est le N - Ac - D - Nal(2) - D - pCl - Phe - D - Trp - Ser - Tyr - D - Deh - Leu - Arg - Pro - D - $AlaNH_2$ ou un sel pharmaceutiquement acceptable de ce composé.

9. Composition suivant l'une quelconque des revendications précédentes, comprenant en outre un surfactant additionnel.

10. Composition suivant l'une quelconque des revendications précédentes, dans laquelle rapport molaire de l'acide biliaire au peptide est supérieur ou égal à 20:1.

11. Procédé de préparation d'un médicament pour favoriser l'absorption nasale d'un analogue de LHRH, qui consiste à ajouter un surfactant qui est un acide biliaire ou un sel pharmaceutiquement acceptable de cet acide à une composition nasale comprenant un nonapeptide ou un décapeptide ou un sel pharmaceutiquement acceptable de ce peptide ayant une activité agoniste ou antagoniste de LHRH, formulé en solution aqueuse.

12. Procédé suivant la revendication 11, qui consiste à ajouter 0,2 à 5% en poids/volume d'un surfactant qui est un acide biliaire ou un sel pharmaceutiquement acceptable de cet acide à une composition nasale pulvérisable comprenant 0,005 à 5 mg/ml d'un nonapeptide ou d'un décapeptide ou de son sel pharmaceutiquement acceptable ayant une activité agoniste ou antagoniste de LHRH; et une solution aqueuse tamponée en une quantité suffisante pour compléter le volume.